# EUROPÄISCHE PATENTSCHRIFT

(11) **EP 0 380 809 B1**
(45) Veröffentlichungstag und Bekanntmachung des Hinweises auf die Patenterteilung: **09.03.1994**
(21) Anmeldenummer: 89124048.3
(22) Anmeldetag: 28.12.1989
(51) Int. Cl.: A61M 1/00, A61B 17/22

(54) **Vorrichtung zum Ein- und Ausbringen von Flüssigkeiten in bzw. aus Körper-Hohlorganen**
Device for introducing and removing liquids, especially into or from body cavities
Appareil pour introduire et retirer des liquides, en particulier de cavités du corps humain

(30) Priorität: 01.02.1989 DE 3902943
(43) Veröffentlichungstag der Anmeldung: 08.08.1990
(73) Patentinhaber: Richard Wolf GmbH, 75438 Knittlingen (DE)
(72) Erfinder: Leuschner, Ulrich, Prof. Dr. med., D-6000 Frankfurt/Main 70 (DE); Ams, Felix, D-7539 Kämpfelbach (DE); Müller, Klaus, D-7134 Knittlingen-Freudenstein (DE)
(74) Vertreter: Wilcken, Thomas, Dipl.-Ing.

(56) Entgegenhaltungen:
- EP-A- 0 214 629
- WO-A-87/00440

## Beschreibung

Die Erfindung betrifft eine Vorrichtung zum Einbringen eines flüssigen Lösungsmittels in ein Körper-Hohlorgan, insbesondere in die Gallenblase, und zum Absaugen der aus Lösungsmittel und Organsekret bestehenden Flüssigkeit, sowie des durch den Auflösungsvorgang entstehenden Steinschlammes aus dem Hohlorgan mittels einer Kolbenpumpe, wobei das Organsekret und das Lösungsmittel im Anschluß daran mit Hilfe eines Abscheiders voneinander trennbar sind.

Für die Durchführung dieser auch unter der Bezeichnung Litholyse bekannten Therapie geeignete Vorrichtungen gehören zum Stand der Technik.

So ist aus der EP-A-214 629 eine Einrichtung mit dem Merkmalen des Oberbegriffs des Anspruchs 1 zu entnehmen, die im wesentlichen aus einer Flüssigkeitspumpe und einem Abscheider besteht. Zwecks Auflösung der Konkremente in der Gallenblase wird in diese eine vorgegebene Menge eines Lösungsmittels eingeleitet, um dieses dort auf die Konkremente lösend einwirken zu lassen. Nach Einwirkung über einen bestimmten Zeitraum wird das Lösungsmittel gemeinsam mit dem sich in der Gallenblase ansammelnden Organsekret mittels durch die Flüssigkeitspumpe erzeugten Unterdruck in den Abscheider gesaugt, wo sich das Organsekret, der Gallensaft, nach und nach im unteren Bereich absetzt, da das Lösungsmittel und der Gallensaft lediglich miteinander dispergieren können. Das darüber befindliche Lösungsmittel kann dann wieder in die Gallenblase zurückgeführt werden, um die Auflösung der Konkremente fortzusetzen. Das Zu- und Abführen des Lösungsmittels wird so oft wiederholt, bis entweder die Konkremente vollständig aufgelöst oder diese durch das Absaugen aus der Gallenblase herausgespült sind. Da während der Litholyse ständig neuer Gallensaft produziert wird, ist es unter Umständen erforderlich, den Abscheider mehrmals während der Therapie zu entleeren.

Der Erfindung liegt die Aufgabe zugrunde, diese bekannte Einrichtung zu vereinfachen und bedienungsfreundlicher zu gestalten.

Diese Aufgabe wird erfindungsgemäß durch die im Kennzeichenenden Teil des Anspruchs 1 genannten Merkmale gelöst.

Die damit erzielbaren Vorteile bestehen insbesondere darin, daß weder ein separater Abscheider noch eine zusätzliche Pumpeneinheit aus Kolben und Zylinder erforderlich sind, da die Kolbenspritze beide Geräteteile ersetzt.

Bei Verwendung einer Kolbenspritze mit einem Verteilerkopf, der mehrere mit dem Druckraum einzeln verbindbare Anschlußkanäle aufweist, ist die Möglichkeit gegeben, bei Bedarf die aus dem Hohlorgan abgesaugte und in dem Druckraum befindliche Flüssigkeit durch einfaches Umschalten durch einen anderen Anschlußkanal hindurch in ein Aufnahmegefäß zu leiten. Über einen dritten Anschlußkanal kann dann auch nach entsprechender erneuter Umschaltung frisches Lösungsmittel aus einem Vorratsbehälter entnommen und so die Kolbenspritze wieder geladen werden, um das Lösungsmittel in einem neuen Zyklus dem Hohlorgan wieder zuzuführen. Diese Handhabung ist möglich, ohne daß dabei ein Ausbau der Kolbenspritze erforderlich ist.

Die erfindungsgemäße Vorrichtung kann auf einfache Weise dadurch automatisiert werden, daß die Kolbenspritze unter senkrechter Ausrichtung ihrer Achse und mit dem Verteilerkopf nach oben weisend in eine elektromotorisch bewegbare Betätigungseinrichtung lösbar einsetzbar ist, wobei auch die vorstehend beschriebenen Arbeitszyklen entsprechend gesteuert werden können dadurch, daß die relative Verdrehung zwischen Verteilerkopf und Rohrkörper durch elektromotorische Verdrehung des Kolbenschaftes erfolgt. Zur Überwachung des Füllungsgrades des Druckraumes der Kolbenspritze, des maximal zulässigen Füllstandes an Organsekret sowie des Strömungsdruckes beim Zuführen des Lösungsmittels in die Gallenblase, können entsprechende Sensoren vorgesehen sein, wobei die Überwachung der erstgenannten Zustände mittels Lichtschranken erfolgen kann.

Für die Gesamtsteuerung kann eine Steuerlogik vorgesehen sein, die die Antriebe zur Verschiebung und Drehung des Kolbens bzw. des Kolbenschaftes unter Berücksichtigung der Steuersignale und einstellbaren Zeitvorgaben sowie des gesamten Funktionsablaufes steuert.

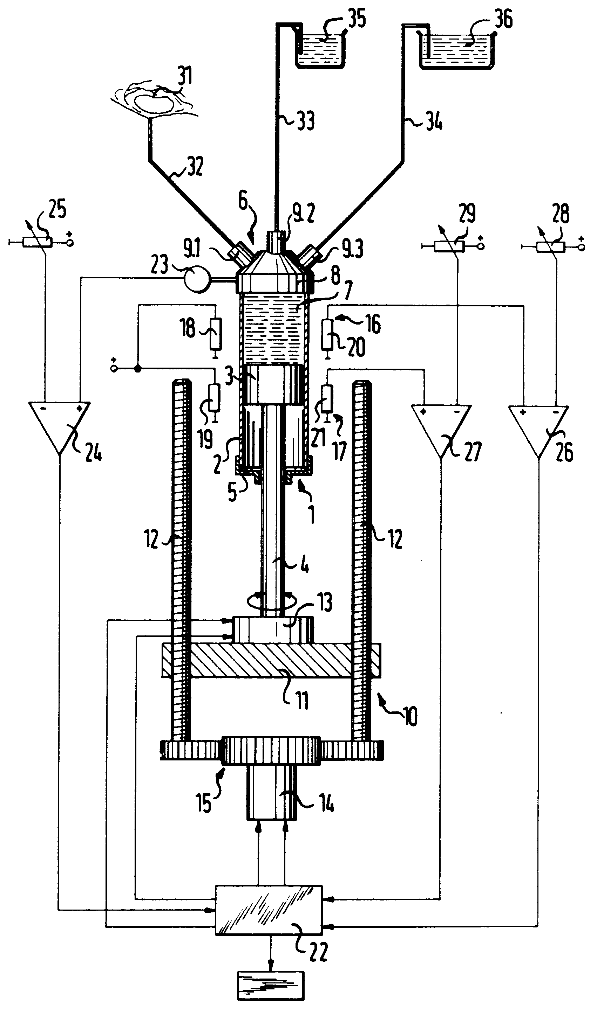

Die Erfindung ist nachfolgend anhand eines in der Zeichnung dargestellten Ausführungsbeispiels näher erläutert.

Die einzige Figur zeigt in schematischer Darstellung die Gesamtanordnung eines solchen Ausführungsbeispieles. Wie in der Darstellung erkennbar, wird das zentrale Element dieser Vorrichtung durch eine Kolbenspritze 1 gebildet, die einen zylindrischen Rohrkörper 2, vorzugsweise aus Glas, einen in diesem gleitdichtend geführten Kolben 3 mit einem Kolbenschaft 4, einen den Rohrkörper 2 an seinem unteren Ende abschließenden und den Kolbenschaft 4 verdrehsicher führenden Führungsflansch 5, einen den Rohrkörper 2 an seinem oberen Ende verschließenden Anschlußflansch 6 und einen von diesem, der Stirnfläche des Kolbens 3 und dem Rohrkörper 2 begrenzten Druckraum 7 aufweist. Der Anschlußflansch 6 ist als Verteilerkopf 8 mit drei Anschlußkanälen 9.1, 9.2 und 9.3 ausgebildet und unter Abdichtung drehbar mit dem Rohrkörper 2 verbunden.

Die Kolbenspritze 1 ist in eine ein nicht gezeigtes Rahmengestell umfassende Betätigungseinrichtung 10 lösbar eingesetzt, wobei der Verteilerkopf 8 in einer ortsfesten Aufnahme festgelegt und das freie Ende des Kolbenschaftes 4 mit einer in zwei Gewindespindeln 12 geführten Kulisse 11 verbunden ist.

Während der Kolbenschaft 4 mit einem denselben um seine Kolbenschaftachse drehbar antreibenden Antriebsmotor 13 gekoppelt ist, sind die Gewindespindeln 12 mittels eines Antriebsmotors 14 über ein Zahnradgetriebe 15 synchron in Rotation versetzbar.

Im Bereich des Druckraumes 7 sind zwei Lichtschranken 16,17 im Abstand zueinander und in einem bestimmten Abstand zur Oberseite des Kolbens 3 längsverschiebbar angeordnet, derart, daß der transparente Rohrkörper 2 zwischen dem jeweiligen Lichtsender 18 bzw. 19 und dem jeweils zugehörigen Lichtempfänger 20 bzw. 21 liegt und von den Lichtstrahlen durchdrungen wird.

Die Vorrichtung umfaßt weiter eine Steuerlogik, bestehend aus einer Motorsteuerung 22, aus einem einen Drucksensor 23 überwachenden Regelkreis aus Operationsverstärker 24 und Regler 25 und aus jeweils einem Regelkreis zur Überwachung der Lichtschranken 16 und 17, bestehend aus Operationsverstärker 26 bzw. 27 und Regler 28 bzw. 29.

Für den Betrieb der erfindungsgemäßen Vorrichtung wird eine mit dem Anschlußkanal 9.1 des Verteilerkopfes 8 verbundene Schlauchleitung 32 in das zu therapierende Hohlorgan, hier beispielsweise die Gallenblase 31, eingeführt und die Anschlußkanäle 9.2 und 9.3 über Schlauchleitungen 33 bzw. 34 mit einem Vorratsbehälter 35 für ein Konkremente auflösendes Lösungsmittel, beispielsweise Methyläther, bzw. mit einem Auffangbehälter 36 zur Aufnahme des Gemisches aus Organsekret, Lösungsmittel und Steinschlamm verbunden. Durch Ingangsetzen des Antriebsmotors 14 kann nun der Kolben 3 in seine obere Endstellung gefahren werden, wobei die Drehstellung des Kolbenschaftes 4 so gewählt wird, daß der Anschlußkanal 9.2 mit dem Druckraum 7 verbunden ist, wodurch die Luft aus diesem durch die Schlauchleitung 33 entweichen kann. Durch Umschalten der Drehrichtung des Antriebsmotors 14 kann darauf der Kolben 3 zurückgezogen werden, was bewirkt, daß Lösungsmittel aus dem Vorratsbehälter 35 durch die Schlauchleitung 33 in den Druckraum 7 eingesaugt wird.

Zwecks Einleitung des ersten Spülzyklus' wird nun dem Funktionsablauf entsprechend durch Aktivierung des Antriebsmotors 13 eine Verbindung des Druckraumes 7 der Kolbenspritze 1 mit dem Anschlußkanal 9.1 hergestellt, der über die Schlauchleitung 32 mit der zu therapierenden Gallenblase 31 korrespondiert. Dieser Umschaltzustand kommt dabei dadurch zustande, daß das auf den Kolbenschaft 4 des Kolbens 3 wirkende Drehmoment des Antriebsmotors 13, aufgrund der Ausbildung des Kolbenschaftes 4 mit beispielsweise quadratischem Querschnitt, auf den mit dem Rohrkörper 2 drehfest verbundenen Anschlußflansch 5 übertragen wird, wodurch der dem drehfest gehalterten Verteilerkopf 8 gegenüber drehbewegliche Rohrkörper 2 eine Drehbewegung ausführt. Alsdann wird der Antriebsmotor 14 in der den Kolben 3 vorschiebender Drehrichtung aktiviert und damit das Lösungsmittel in die Gallenblase 31 eingebracht.

Nach Ablauf einer voreingestellten Einwirkzeit des Lösungsmittels in der Gallenblase 31 wird der Kolben 3 wieder zurückgezogen, wobei der dadurch in dem Druckraum 7 entstehende Unterdruck bewirkt, daß die Gallenblase 31 entleert wird. Dabei gelangt das zuvor in die Gallenblase geleitete Lösungsmittel gemeinsam mit inzwischen produziertem Gallensaft sowie Steinschlamm in den Druckraum 7. Diese Flüssigkeiten bilden eine Dispersion, die sich nach einer gewissen Ruhezeit entmischt. Der Kolben 3 wird daher während einer entsprechenden, voreinstellbaren Absetzzeit in der Rückzugstellung belassen, so daß sich die einzelnen Bestandteile entsprechend ihrer Dichte abzusetzen vermögen. Dabei verbleibt das die geringste Dichte aufweisende Lösungsmittel im Kopfbereich des Druckraumes 7, so daß bei einem erneuten Zyklus im wesentlichen nur Lösungsmittel, allenfalls mit geringen Mengen Gallensaft in die Gallenblase 31 zurückgelangt.

Der Tatsache, daß sich die gesamte Flüssigkeitsmenge von einem Spülzyklus zum nächsten durch die ständige Neubildung von Gallensaft und durch die Zunahme des Steinschlamms vergrößert, wird beim Absaugen der Flüssigkeit aus der Gallenblase in den Druckraum 7 dadurch Rechnung getragen, daß der Hub des Kolbens 3(linear) mit der Anzahl der durchfahrenen Zyklen entsprechend vergrößert wird. Der beschriebene Zyklus wird so lange wiederholt, bis die Konkremente aufgelöst bzw. die restlichen Konkremente aufgrund der wechselnden Strömungsrichtung und Druckphasen ausgespült sind.

Da sich von Zyklus zu Zyklus eine Anreicherung des Lösungsmittels mit Gallensaft und Steinschlamm ergibt, erfolgt eine Überwachung des Volumenanteils des sich in dem Druckraum 7 der Kolbenspritze 1 während der Therapie ansammelnden Gallensaftes und Steinschlammes mittels der Lichtschranke 17. Diese erzeugt ein Signal, das als den aktuellen Volumenanteil repräsentierender Istwert einem Vergleicher 27 zugeführt wird. Dem zweiten Eingang des Vergleichers 27 wird ein den höchstzulässigen Volumenanteil von Gallensaft und Steinschlamm repräsentierender Maximalwert zugeführt, welcher mittels eines Reglers 29 voreinstellbar ist.

Das Ausgangssignal des Vergleichers 27 greift bei Gleichstand zwischen dem erwähnten Istwert und Maximalwert in die Regelung der Motorsteuerung 22 ein. Dieser Eingriff kann sich dann so auswirken, daß zunächst die Zyklusfolge unterbrochen und stattdessen der Antriebsmotor 13 aktiviert wird, derart, daß eine Verbindung des Druckraumes 7 mit dem Auffangbehälter 36 über den Anschlußkanal 9.3 und die Schlauchleitung 34 hergestellt wird. Anschließend wird der Kolben 3 wieder vorgeschoben, auf welche Weise der Inhalt der Kolbenspritze 1 in den Auffangbehälter 36 gelangt. Durch erneute Aktivierung des Antriebsmotors 13 wird dann eine Verbindung des Druckraumes 7 mit dem Vorratsbehälter 35 geschaffen und in dem oben beschriebenen Vorgang neues Lösungsmittel übernommen, um die Therapie fortzusetzen.

Während der Austauschvorgänge kann es vorkommen, daß der freie Querschnitt der Schlauchleitung 32 durch Steinschlamm oder Konkrementteile verlegt wird. Die sich daraus ergebenden Druck- bzw. Unterdrucküberhöhungen werden durch den Drucksensor 23 registriert, dessen Maximaldruck an dem Regler 25 eingestellt werden kann, wobei wiederum bei Gleichstand zwischen Sollwert und Istwert an dem Operationsverstärker 24 durch diesen ein Ausgangssignal erzeugt wird, welches das sofortige Abschalten des Antriebsmotors 14 bewirkt, um eventuelle Beschädigungen zu vermeiden, und ein optisches und/oder akustisches Warnsignal auslöst.

## Patentansprüche

1. Vorrichtung zum Einbringen eines flüssigen Lösungsmittels in ein Körper-Hohlorgan, insbesondere in die Gallenblase, und zum Absaugen der aus Lösungsmittel und Organsekret bestehenden Flüssigkeit sowie des durch einen Auflösungsvorgang entstehenden Steinschlammes aus dem Hohlorgan mittels einer Kolbenpumpe (1), wobei das Organsekret und das Lösungsmittel anschließend mit Hilfe eines Abscheiders (7) voneinander trennbar sind, dadurch gekennzeichnet, daß der Abscheider durch den Druckraum (7) einer senkrecht ausgerichteten Kolbenpumpe (1) gebildet ist, daß der durch einen Rohrkörper (2) geformte Druckraum (7) oben durch einen Verteilerkopf (8) mit mehreren Anschlußkanälen (9.1, 9.2, 9.3) begrenzt ist, daß der Rohrkörper (2) und der Verteilerkopf (8) zum Anschließen der Anschlußkanäle relativ zueinander verdrehbar sind und daß die Kolbenpumpe (1) unter senkrechter Ausrichtung ihrer Achse in eine elektromotorisch antreibbare Betätigungseinrichtung (10) eingesetzt ist.

2. Vorrichtung nach Anspruch 1, dadurch gekennzeichnet, daß ein erster Sensor (16) zur Überwachung des maximal zulässigen Füllungsgrades des Druckraumes (7), ein zweiter Sensor (17) zur Überwachung des maximal zulässigen Füllstandes des Organsekretes sowie ein weiterer Sensor (23) zur Überwachung des Strömungsdruckes beim Zuführen des Lösungsmittels in die Gallenblase vorgesehen ist.

3. Vorrichtung nach Anspruch 2, dadurch gekennzeichnet, daß die Sensoren (16 und 17) jeweils als Lichtschranken (17, 20 bzw. 19, 21) ausgebildet sind.

4. Vorrichtung nach einem der vorhergehenden Ansprüche, dadurch gekennzeichnet, daß eine Steuerlogik (22-29) zur Steuerung des den Pumpenkolben (3) in Längsrichtung verschiebenden Antriebsmotors (14) der Betätigungseinrichtung (10) und eines den Rohrkörper (2) über den Kolbenschaft (4) der Kolbenpumpe (1) verdrehenden Antriebsmotors (13) unter Berücksichtigung der Sensorsignale und einstellbarer Zeitvorgaben sowie des gesamten Funktionsablaufes vorgesehen ist.

5. Vorrichtung nach einem der vorhergehenden Ansprüche, dadurch gekennzeichnet, daß die Kolbenpumpe (1) eine handelsübliche Kolbenspritze mit Dreiwegeventil ist.

## Claims

1. Device for introducing a liquid solvent into a hollow body organ, in particular into the gall bladder, and for aspirating the fluid consisting of solvent and organ secretion and the stone sludge being produced by a dissolution process from the hollow organ by means of a piston pump (1), it being possible for the organ secretion and the solvent to then be separated from one another with the aid of a separator (7), characterised in that the separator is formed by the pressure chamber (7) of a vertically aligned piston pump (1), in that the pressure chamber (7) formed by a tubular body (2) is defined at the top by a distribution head (8) having several connecting channels (9.1, 9.2, 9.3), in that the tubular body (2) and the distribution head (8) can be rotated relative to one another to connect the connecting channels, and in that the piston pump (1) is inserted in an electromotively drivable actuation device (10) with vertical alignment of it axis.

2. Device according to claim 1, characterised in that a first sensor (16) is provided to monitor the maximum permissible degree of admission of the pressure chamber (7), a second sensor (17) for monitoring the maximum permissible filling level for the organ secretion, and a further sensor (23) for monitoring the flow pressure when introducing the solvent into the gall bladder.

3. Device according to claim 2, characterised in that the sensors (16 and 17) are each designed as light barriers (17, 20 or 19, 21).

4. Device according to one of the preceding claims, characterised in that control logic (22-29) is provided to control the drive motor (14) of the actuation device (10) displacing the pump piston (3) in the longitudinal direction and a drive motor (13) rotating the tubular body (2) via the piston shaft (4) of the piston pump (1) while taking into account the sensor signals and adjustable rate settings as well as the entire functional course.

5. Device according to one of the preceding claims, characterised in that the piston pump (1) is a commercially available piston plunger with three-way valve.

## Revendications

1. Dispositif permettant d'introduire un solvant liquide dans un organe creux du corps humain, en particulier dans la vésicule biliaire, et pour en aspirer le liquide constitué de solvant et de la sécrétion de l'organe, ainsi que de la boue de calculs formée au cours de la dissolution de l'organe creux à l'aide d'une pompe à piston (1), la sécrétion de l'organe et le solvant pouvant être séparés l'un de l'autre ensuite à l'aide d'un séparateur (7), caractérisé en ce que le séparateur est formé par la chambre sous pression (7) d'une pompe à piston (1) agencé verticalement, la chambre sous pression (7) formée par un corps tubulaire (2) est limitée au-dessus par une tête de distribution (8) avec plusieurs canaux de raccordement (9.1, 9.2, 9.3), le corps tubulaire (2) et la tête de distribution (8) peuvent tourner l'une par rapport à l'autre afin de raccorder les canaux de raccordement et la pompe à piston (1) est installée perpendiculairement à son axe dans un dispositif de commande (10) qui peut être entraîné par un moteur électrique.

2. Dispositif selon la revendication 1, caractérisé en ce qu'un premier capteur (16) est prévu pour contrôler le degré de remplissage maximum autorisé de la chambre sous pression (7), un deuxième capteur (17) est prévu pour contrôler l'état de remplissage maximum autorisé de sécrétion d'organe et un autre capteur (23) est prévu pour contrôler la pression d'écoulement lorsque l'on achemine le solvant dans la vésicule biliaire.

3. Dispositif selon la revendication 2, caractérisé en ce que les capteurs (16 et 27) se présentent respectivement sous la forme de barrières photo-électriques (17, 20 ou 19, 21).

4. Dispositif selon l'une quelconque des revendications précédentes, caractérisé en ce qu'un circuit logique de commande (22-29) est prévu pour commander le moteur d'entraînement (14) du dispositif de commande (10) déplaçant le piston de pompe (3) dans la direction longitudinale et un moteur d'entraînement (13) faisant tourner le corps tubulaire (2) sur la tige de piston (4) de la pompe (1) en tenant compte des signaux des capteurs et des périodes de temps prédéterminées réglables ainsi que du déroulement complet du fonctionnement.

5. Dispositif selon l'une quelconque des revendications précédentes, caractérisé en ce que la pompe à piston (1) est un injecteur à piston du commerce avec une soupape à trois voies.
